# EUROPÄISCHE PATENTSCHRIFT

(11) **EP 2 380 491 B1**
(45) Veröffentlichungstag und Bekanntmachung des Hinweises auf die Patenterteilung: **10.06.2020**
(21) Anmeldenummer: 11156534.7
(22) Anmeldetag: 02.03.2011
(51) Int. Cl.: A61B 5/0215

(54) **Tragbare Sensorvorrichtung und Patientenmonitor**
Portable sensor device and patient monitor
Dispositif de capteur portatif et moniteur de patient

(30) Priorität: 08.03.2010 DE 102010010610
(43) Veröffentlichungstag der Anmeldung: 26.10.2011
(73) Patentinhaber: Pulsion Medical Systems AG, 81829 München (DE)
(72) Erfinder: Steiner, Christian, Dr., 81829, München (DE); Gutzler, Dominik, 90425, Nürnberg (DE); Veeck, Marcus, 56072, Koblenz (DE); Scheier, Jörg, Dr., 80639, München (DE); Manegold, Christoph, 81825, München (DE)
(74) Vertreter: Ascherl, Andreas

(56) Entgegenhaltungen:
- DE-U1- 9 408 557
- US-A- 5 711 302
- US-A- 5 758 657
- US-A1- 2007 179 400
- US-A1- 2008 281 168
- "PiCCOplus short setup", , 1. April 2007 (2007-04-01), Seiten 1-12, XP55000540, Gefunden im Internet: URL:http://www.pulsion.de/fileadmin/pulsio n_share/Products/PiCCO/US/PiCCOplus_ShortS etup_US_MPI812905US_R00_190407.pdf [gefunden am 2011-06-10]

## Beschreibung

Die vorliegende Erfindung wird durch die angehängten Ansprüche definiert.

Tragbare Sensorvorrichtungen und zugehörige Patientenmonitore sind aus der klinischen Anwendung vielfach bekannt. Insbesondere seit langem verbreitet sind Patientenmonitore, welche einen intravasal mittels eines arteriellen Katheters gemessenen Druck überwachen. Dabei wird der Flüssigkeitsdruck am Messort über einen Druckschlauch zu einer tragbaren Sensorvorrichtung übertragen, welche im wesentlichen aus einem eingehausten elektronischen Drucksensor sowie einem Stecker zur Ausgabe des Sensorsignals über ein geeignetes Kabel an den Patientenmonitor besteht.

Entsprechende Sensorvorrichtungen und Patientenmonitore sind beispielsweise unter der Marke PiCCO der Patentanmelderin PULSION Medical Systems AG kommerziell verfügbar. Ferner sind derartige Sensorvorrichtungen und Patientenmonitore auch aus US 5,758,657 und US 2008/0281168 A1 bekannt, wobei letztere einen tragbaren Patientenmonitor offenbart. Auch US 5,711,302 offenbart einen tragbaren Patientenmonitor, wobei hier ein Drucksensor in einen einfachen tragbaren Druckmessmonitor integriert ist. DE 94 08 557 U1 offenbart einen an medizinische Katheter anschließbaren Drucksensor, der zur Vornahme einer Nullmessung umgeschaltet werden kann, und einem angeschlossenen Druckmessmonitor ein Signal übermittelt, welches die Vornahme der Nullmessung anzeigt.

US 2007/179400 A1 offenbart einen medizinischen Drucksensor für die Urologie, insbesondere zur Messung des Blasendrucks, sowie für die rektale Messung des intraabdominalen Drucks, wobei ein das Sensorgehäuse ein Chip integriert ist, welcher verschiedene Funktionen übernehmen kann, z.B. eine Begrenzung der Nutzungsdauer des Sensors. Das Sensorbauteil ist über eine Steckverbindung mit einem elektronischen Bauteil verbindbar, welches als Schnittstelle zu einem Patientenmonitor dient.

Im klinischen Einsatz tragbarer Sensorvorrichtungen und zugehöriger Patientenmonitore der eingangs genannten Art können sich Probleme aufgrund des Umstands ergeben, dass das klinische Personal zusätzlich dadurch beansprucht ist, im Zusammenhang mit der Nutzung der Sensorvorrichtung stehende oder gar für deren Nutzung erforderliche Daten in den Patientenmonitor einzugeben. Hierbei kann es sich beispielsweise um Patientendaten, Kalibrierdaten, Sensortypdaten usw. handeln. Derartige Eingaben beanspruchen Zeit und Aufmerksamkeit des klinischen Personals, welche dann in entsprechend reduziertem Maß für medizinische Maßnahmen am Patienten zur Verfügung stehen. Der für die Dateneingaben erforderliche Zeitaufwand und die damit einhergehende Ablenkung mögen zwar oftmals zu verschmerzen sein, aus prinzipiellen Gründen steigern sie jedoch die Fehlerwahrscheinlichkeit und zwar in umso höherem Maße, je häufiger die Dateneingaben zu erfolgen haben.

Fehlerhafte Dateneingaben können zudem zu fehlerhaften Diagnoseergebnissen führen, wenn physiologische Parameter beispielsweise auf ein falsches Patientengewicht normiert werden.

Häufig durchläuft ein Patient in einer Klinik mehrere Stadien, welche jeweils die Überwachung physiologischer Größen mittels Patientenmonitoren erfordern, beispielsweise Notaufnahme, präoperativen Aufenthalt auf einer Intensivstation, chirurgische Behandlung im Operationssaal und postoperativen Aufenthalt auf einer Intensivstation. Ist jedesmal neuerlich die Eingabe umfangreicher Daten in den jeweiligen Patientenmonitor erforderlich, so potenziert sich die obige Problematik.

Vor dem Hintergrund der obigen Problematik ist es Aufgabe der vorliegenden Erfindung, eine tragbare Sensorvorrichtung und einen zugehörigen Patientenmonitor bereitzustellen, welche den Aufwand für Dateneingaben vermindern.

Ferner besteht in der Praxis oft die Anforderung, zwei Monitore (etwa ein Patientenmonitor zur Ermittlung hämodynamischer Parameter mittels Pulskonturanalyse und einen einfacheren Blutdruckmonitor zugleich zu betreiben. Hierfür sind bislang zwei separate Drucklinien zum Patienten zu legen, was einen entsprechenden Aufwand bedeutet und zudem die Anzahl der am Patienten applizierten Anschlüsse erhöht. Es ist vor diesem Hintergund weiter Aufgabe der Erfindung, den geleichzeitigen Betrieb eines Patientenmonitors und eines einfachen Blutdruckmonitor zu vereinfachen.

Dateneingaben am Patientenmonitor dienen oft dem Zusammenspiel von Patientenmonitor und Sensorvorrichtung. Dieses Zusammenspiel lässt sich erfindungsgemäß sowohl durch Übertragung einer über das reine Sensorsignal hinausgehenden Information (beispielsweise einer Sensortypkennung oder eines Kalibrierwerts) von der Sensorvorrichtung zum Patientenmonitor, als auch durch Übertragung einer Information vom Patientenmonitor zu der Sensorvorrichtung verbessern.

Gemäß einem Aspekt der vorliegenden Erfindung wird die zugrunde liegende Aufgabe gelöst durch eine tragbare Sensorvorrichtung gemäß Anspruch 1.

Bei dem Datensignal handelt es sich vorzugsweise um ein digitales Signal, jedoch ist die Erfindung vorteilhaft auch mit (beispielsweise moduliertem) analogem Datensignal realisierbar.

Entsprechend wird die zugrunde liegende Aufgabe gemäß einem weiteren Aspekt der vorliegenden Erfindung durch ein System gemäß Anspruch 7gelöst.

Vorzugsweise umfasst das mindestens eine elektronische Zusatzbauteil einen Speicher zum Speichern von Patientendaten. So können Patientendaten, wie etwa Alter, Geschlecht, Körpergröße oder Körpergewicht sowie ggf. hiervon abgeleitete Größen wie Body Mass Index, spezifische Körperoberfläche etc., welche zuvor in den Patientenmonitor eingegeben oder von diesem errechnet wurden, zur Sensorvorrichtung ausgelesen werden. Durchläuft ein Patient in einer Klinik mehrere Stadien, beispielsweise Notaufnahme, präoperativen Aufenthalt auf einer Intensivstation, chirurgische Behandlung im Operationssaal und postoperativen Aufenthalt auf einer Intensivstation, so müssen nicht jedesmal neuerlich Daten in den jeweiligen Patientenmonitor eingegeben werden. Vielmehr genügt die Eingabe der Daten beim ersten zum Einsatz kommenden Patientenmonitor. Die tragbare Sensorvorrichtung kann dann als eine Art elektronische Patienten-ID beim Patienten (z.B. an einer patientenbettseitigen Halterung, an einem Oberarm- oder Brustband des Patienten oder dgl.) bleiben, und die Daten jedem weiteren zum Einsatz kommenden Patientenmonitor zur Verfügung stellen. Patientendaten der genannten Art werden von Patientenmonitoren mitunter benötigt, um ermittelte physiologische Parameter auf geeignete Weise normieren zu können, oder aber um gewisse diagnoseunterstützende Ausgaben zu erlauben, d.h. beispielsweise einen Alarm auszugeben, wenn ein ermittelter Parameter einen kritischen Wert annimmt oder unter den gegebenen Umständen nicht zuverlässig berechnet werden kann. So können etwa für pädiatrische und geriatrische Patienten völlig unterschiedliche Grenzwerte gelten, ab welchen ein bestimmter hämodynamischer Parameter als kritisch anzusehen ist, oder aber gewisse Parameter gewinnen erst diagnostische Aussagekraft, wenn sie auf die Körpergröße, das Körpergewicht, den Body-Mass-Index oder dgl. normiert sind.

Während die zuletzt geschilderte spezielle Ausführungsform einen Kanal zur Ausgabe der gespeicherten Daten an weitere Patientenmonitoren erfordert, kann die Übertragung einer Information vom Patientenmonitor zu der Sensorvorrichtung die Beanspruchung des klinischen Personals auch mindern, wenn kein Kanal zur Ausgabe in der Sensorvorrichtung gespeicherter Daten vorgesehen ist. So können etwa, wie nachfolgend erläutert, patientendatenabhängige Warnfunktionen statt im Patientenmonitor in der Sensorvorrichtung implementiert werden. Oder aber Anpassungsfunktionen zur korrekten Kommunikation zwischen Patientenmonitor und Sensorvorrichtung werden sensorseitig statt monitorseitig vorgenommen (z.B. eine monitoranschlussspezifische Anpassung der Ausgangsimpedanz der Sensorvorrichtung statt einer sensoranschlussspezifischen Anpassung der Eingangsimpedanz des betreffenden Patientenmonitoranschlusses.)

Gemäß einer vorteilhaften Ausführungsform weist die Sensorvorrichtung eine optische und/oder akkustische Ausgabevorrichtung auf. Vorteilhafterweise kann es sich hierbei zum Beispiel um eine oder mehrere Leuchtdioden, eine kleine Flüssigkristallanzeige, einen elektronischen Klangerzeugerchip mit zugeordnetem Lautsprecher oder dergleichen handeln. Je nach elektronischer Ausstattung der Sensorvorrichtung kann eine solche Ausgabevorrichtung von entsprechenden Komponenten der Sensorvorrichtung oder vom Patientenmonitor direkt über den Eingangskanal der Sensorvorrichtung angesteuert werden. So lassen sich vorteilhafterweise verschiedene Alarmfunktionen umsetzen, etwa eine Warnung bei über oder Unterschreitung eines kritischen Schwellenwerts eines physiologischen Parameters, bei Kommunikationsproblemen zwischen Patientenmonitor und Sensorvorrichtung oder anderen technischen Störungen etc. Neben reinen Alarmfunktionen sind auch komplexere Anzeigefunktionen vorteilhaft unsetzbar, etwa die Anzeige von Patientendaten auf einem Flüssigkristalldisplay. Derartige Ausgabevorrichtungen können aber müssen nicht mit in das Sensorgehäuse intergriert sein, sondern können beispielsweise auf einem steckbaren Zusatzmodul oder dergleichen untergebracht sein.

Grundsätzlich kann für das elektronische Sensorbauteil per se bereits bekannte Sensortechnik, d.h. beispielsweise kapazitive Membrandruckaufnehmer oder Piezodruckaufnehmer, vorgesehen sein.

Erfindungsgemäß lassen sich an einer Drucklinie zwei Drucksensoren anschließen, d.h. die erfindungsgemäße Sensorvorrichtung lässt sich beispielsweise zwischen eine herkömmliche Sensorvorrichtung und einen Druckmesskatheter schalten, so dass zwei Monitore (etwa ein Patientenmonitor zur Ermittlung hämodynamischer Parameter mittels Pulskonturanalyse und ein einfacherer Blutdruckmonitor) zugleich mit jeweils kompatiblem Sensor betrieben werden können.

Vorzugsweise ist die tragbare Sensorvorrichtung in der Richtung Ihrer größten Ausdehnung kleiner als 100 Millimeter und leichter als 100 Gramm, was insbesondere dann von Vorteil ist, wenn die Sensorvorrichtung über mehrere Stationen am oder beim Patienten verbleiben soll, etwa um die oben beschriebene Übertragung von Patientendaten auf wechselnde Patientenmonitore zu ermöglichen. Zur patientenbettseitigen Anbringung besitzt das Sensorgehäuse vorzugsweise einen mechanischen Halterungsanschluss, der es ermöglicht, die Sensorvorrichtung, vorzugsweise werkzeugfrei, an einer patientenbettseitigen Halterung anzubringen und, ebenfalls vorzugsweise werkzeugfrei, von dieser zu lösen. Gemäß einer besonders vorteilhaften Ausführungsform ist der Halterungsanschluss mit einem Adaptersystem kombinierbar, welches den Anschluss an unterschiedliche Halterungen ermöglicht, so dass insbesondere die Konnektivität zu unterschiedlichen in der klinischen Praxis verwendeten Halteplattensystemen gewährleistet ist.

Die Sensorvorrichtung ist steril, um auch im OP-Bereich eingesetzt werden zu können. Hierfür wird die tragbare Sensorvorrichtung vorteilhafterweise vor Gebrauch sterilverpackt bereitgehalten.

Gemäß einer besonders bevorzugten Ausführungsform ist die Patientenmonitorschnittstelle zum Herstellen einer analogen Signalverbindung zwischen dem elektronischen Sensorbauteil und dem Patientenmonitor zum Übermitteln eines analogen Sensorsignals eingerichtet, die tragbare Sensorvorrichtung weist ein elektronisches Kennzeichnungsbauteil zum Bereitstellen eines Kennzeichnungssignals, welches eine Eigenschaft des durch die analoge Signalverbindung zu übermittelnden Sensorsignals kennzeichnet, auf, und die Patientenmonitorschnittstelle weist einen separaten Kennzeichnungskanal zum Ausgeben des Kennzeichnungssignals an den Patientenmonitor auf.

Vorzugsweise ist die Patientenmonitorschnittstelle zum Herstellen einer analogen Signalverbindung zwischen dem elektronischen Sensorbauteil und dem Patientenmonitor zum Übermitteln eines analogen Sensorsignals ausgebildet. Dabei besitzt die tragbare Sensorvorrichtung ein elektronisches Kennzeichnungsbauteil zum Bereitstellen eines Kennzeichnungssignals, welches eine Eigenschaft des durch die analoge Signalverbindung zu übermittelnden Sensorsignals kennzeichnet, und die Patientenmonitorschnittstelle einen separaten Kennzeichnungskanal zum Ausgeben des Kennzeichnungssignals an den Patientenmonitor.

So können beispielsweise in der Sensorvorrichtung gespeicherte Kalibrierdaten an den Patientenmonitor übermittelt werden, um eine korrekte Sensorsignalauswertung zu ermöglichen. Auch sind so vorteilhaft flexible Anschlusssysteme realisierbar, bei welchen mehrere und/oder verschiedene Sensoren an einen Patientenmonitor anschließbar sind, und dieser erkennt, wie das jeweilige Signal zu verarbeiten ist.

Bei dem Kennzeichnungssignal handelt es sich vorzugsweise um ein digitales Signal, jedoch ist die Erfindung vorteilhaft auch mit (beispielsweise moduliertem) analogem Kennzeichnungssignal realisierbar.

Bei einem entsprechenden vorteilhaften Patientenmonitor ist die Sensorschnittstelle zum Anschließen von Patientenmonitorverbindungsmitteln zum Herstellen einer analogen Signalverbindung zwischen einer solchen tragbaren Sensorvorrichtung und dem Patientenmonitor ausgebildet, und der Patientenmonitor weist einen separaten Erkennungskanal zum Einlesen des Kennzeichnungssignals in den Patientenmonitor auf. Der Patientenmonitor ist dazu ausgebildet, das durch die analoge Signalverbindung zu übermittelnde Sensorsignal in Abhängigkeit des Kennzeichnungssignals zu verarbeiten.

Beispielsweise kann ein solcher Patientenmonitor mit mehreren Sensoranschlüssen versehen sein, wobei es gleichgültig ist, mit welchem Sensoranschluss eine bestimmte Sensorvorrichtung verbunden ist, weil der Patientenmonitor das entsprechende Kennzeichnungssignal erkennt, und das über den entsprechenden Sensoranschluss eingehende Sensorsignal richtig zuordnet und weiterverarbeitet.

Gemäß einer besonders vorteilhaften Weiterbildung der Erfindung ist das Kennzeichnungsbauteil mit Nullerkennungsmitteln zum Erkennen eines Nullabgleichs ausgestattet, und das Kennzeichnungssignal kennzeichnet das Sensorsignal als Nullsignal. Vorteilhafterweise kann eine solche Funktion zur Kennzeichnung des Sensorsignals als Nullsignal kombiniert sein mit anderweitigen Kennzeichnungssignalen zur Übermittlung des Sensortyps oder dergleichen.

Die Nullerkennungsmittel können unterschiedlich realisiert werden. Ist das elektronische Sensorbauteil einer erfindungsgemäßen Sensorvorrichtung als Drucksensor ausgebildet, und besitzt die Sensorvorrichtung eine manuell bedienbare Ventilvorrichtung (z.B. Dreiwegehahn) zum Nullen mit Atmosphärendruck, so kann die Ventilvorrichtung mit einem Schaltkontakt versehen sein, der eine Ventilstellung erkennt, bei welcher eine Verbindung zur Atmosphäre hergestellt ist. Alternativ kann in der Sensorvorrichtung ein Mikrokontroller, DSP oder dgl. vorgesehen sein, welcher einen für eine Nullmessung bzw. Kalibriermessung typischen Signalverlauf erkennt, beispielsweise einen plötzlichen und/oder über einen gewissen Wert hinausgehenden Druckabfall mit anschließend innerhalb vorgegebener Grenzen konstanten Druck.

Ferner kann eine Sensorvorrichtung mit einem Schaltknopf oder einer anderen manuellen Schaltvorrichtung ausgestattet sein, welche bei Vornahme einer Nullmessung (d.h. Kalibriermessung) manuell zu betätigen ist. Gegenüber einer Eingabe am Patientenmonitor hat dies den Vorteil einer wesentlich vereinfachten Handhabung, da bei der Vornahme einer Kalibriermessung so nur mit der Sensorvorrichtung, nicht aber mit Sensorvorrichtung und Patientenmonitor zugleich zu hantieren ist.

Nachfolgend werden anhand der zugehörigen Zeichnungen Beispiele bevorzugter Ausführungsformen der vorliegenden Erfindung näher erläutert.

Die Zeichnungen sind dabei schematische und, aus Gründen der Anschaulichkeit, nicht exakt maßstäbliche Darstellungen. Insbesondere können Verhältnisse der Abmessungen zueinander von tatsächlichen Ausführungen, zum Teil auch stark, abweichen.

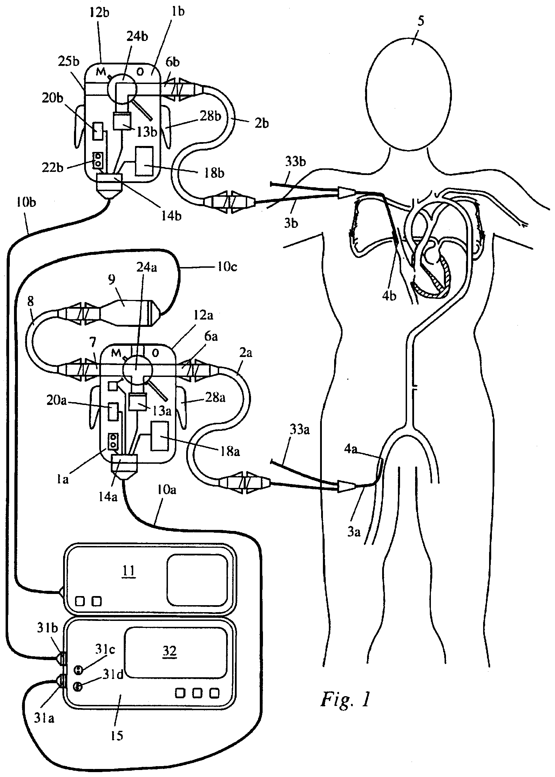

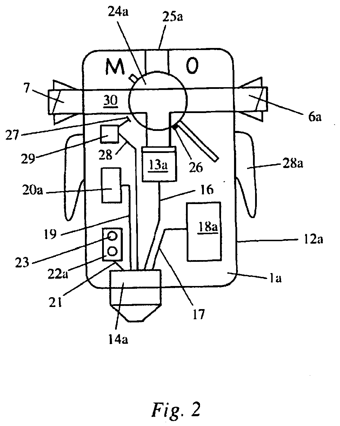

In beiden Figuren dargestellte Elemente sind in den einzelnen Figuren jeweils mit den gleichen Bezugszeichen versehen. Aus Gründen der Übersichtlichkeit tragen jedoch in Figur 1 nicht alle der in Figur 2 ebenfalls zu sehenden Elemente Bezugszeichen. Es zeigt
- Fig. 1: zwei erfindungsgemäße Sensorvorrichtungen in einer Anordnung zusammen mit einem zugehörigen Patientenmonitor, einem weiteren Sensor sowie einem an den weiteren Sensor angeschlossenen zusätzlichen Blutdruckmonitor,
- Fig. 2: eine vergrößerte schematische Darstellung eines der Fig. 1 dargestellten Sensorvorrichtungen.

Die tragbare Sensorvorrichtung 1a ist über einen Druckschlauch 2a mit dem arteriellen Katheter 3a zur Messung des arteriellen Drucks an einem Messort 4a in der Femoralarterie des Patienten 5 verbunden. Der Druckschlauch 2a ist über die als Luer-Lock-Verbindung ausgeführte Messortschnittstelle 6a an der Sensorvorrichtung 1a angeschlossen. Im Sensorgehäuse 12a der Sensorvorrichtung 1a ist der elektronische Drucksensor 13a untergebracht. Das analoge Sensorsignal des Drucksensors 13a wird über ein an die Patientenmonitorschnittstelle 14a angeschlossenes Kabel 10a an den Patientenmonitor 15 ausgegeben.

Neben dem Kanal 16 für das analoge Sensorsignal sind weitere Kanäle zur Kommunikation zwischen Sensorvorrichtung 1a und Patientenmonitor 15 vorgesehen. Ein bidirektionaler Kanal 17 dient dem Beschreiben und Abfragen des Speicherbausteins 18a, in welchem Patientendaten gespeichert werden können. Über den Kennzeichnungskanal 19 kann ein Kennzeichnungssignal von dem Kennzeichnungsbauteil 20a ausgelesen werden. Bei dem Kennzeichnungssignal handelt es sich vorteilhafterweise um eine Sensortypenkennung, welche es dem Patientenmonitor 15 ermöglicht, zu erkennen, um welche Sensorvorrichtung 1a es sich handelt. Das Kennzeichnungssignal kann auch auf eine fälschungssichere Weise codiert sein (z.B. mittels eines per se bekannten Verschlüsselungsverfahrens), wodurch sich sicherstellen lässt, dass nur geeignete Sensorvorrichtungen 1a, 1b zusammen mit dem Patientenmonitor 15 betrieben werden können, indem dieser nur Sensorsignale von Sensorvorrichtungen 1a, 1b akzeptiert, welche ein korrekt codiertes Kennzeichnungssignal bereitstellen. Der unter Umständen patientengefährdende Betrieb nicht zugelassener Sensoren kann so sicher vermieden werden. Über einen weiteren Kanal 21 kann eine Ausgabevorrichtung 22a mit zwei Leuchtdioden 23 angesteuert werden. Warnsignale des Patientenmonitors, z.B. wenn ein ermittelter physiologischer Parameter einen kritischen Wert annimmt, können so, beispielsweise über eine rote Leuchtdiode 23, patientennah ausgegeben werden. Eine weiter, beispielsweise grüne, Leuchtdiode kann anzeigen, dass die Verbindung zwischen Patientenmonitorschnittstelle 14a und Sensorschnittstelle des Patientenmonitors 15 über das Kabel 10a ordnungsgemäß hergestellt ist.

Die Sensorvorrichtungen 1a besitzt eine Ventilvorrichtung 24a, mit welcher der Drucksensor 13a gegen Atmosphärendruck kalibriert, also genullt werden kann. Hierfür ist eine Kalibrieröffnung 25a vorgesehen. Die Ventilvorrichtung 24a umfasst einen Dreiwegehahn mit den Stellungen "M" (Messbetrieb) und "0" (Kalibriermessung). Der Kontaktgeber 26 wird in der Stellung "0" in Verbindung mit dem Kontakt 27 gebracht. Der so hergestellte Schaltkontakt wird von dem (beispielsweise kapazitiv messenden) Detektor 29 detektiert und ein entsprechendes Signal über den Kanal 28 an den Patientenmonitor 15 übertragen. Auf diese Weise wird das Nullen der Sensorvorrichtung 1a dem Patientenmonitor 15 automatisch angezeigt.

Mittels des an der Rückseite des Sensorgehäuses 12a aufgesteckten Halteplattenadapters 28a kann die Sensorvorrichtung 1a werkzeugfrei an einer patientenbettseitigen Halteplatte angebracht und wieder werkzeugfrei von dieser gelöst werden.

Über die ebenfalls als Luer-Lock-Verbindung ausgeführte Drucklinienschnittstelle 7 und einen weiteren Druckschlauch 8 besteht eine hydraulische Verbindung 30 von der Messortschnittstelle 14a zum zusätzlichen, herkömmlichen Drucksensor 9, welcher über das Kabel 10c am Blutdruckmonitor 11 angeschlossen ist.

An den Patientenmonitor 15 ist ferner eine zweite erfindungsgemäße Sensorvorrichtung 1b angeschlossen. Die tragbare Sensorvorrichtung 1b ist über einen Druckschlauch 2b mit dem zentralvenösen Katheter 3b zur Messung des zentralvenösen Drucks an einem Messort 4b in der Vena cava superior des Patienten 5 verbunden. Der Druckschlauch 2b ist über die als Luer-Lock-Verbindung ausgeführte Messortschnittstelle 6b an der Sensorvorrichtung 1b angeschlossen. Im Sensorgehäuse 12b der Sensorvorrichtung 1a ist der elektronische Drucksensor 13b untergebracht. Das analoge Sensorsignal des Drucksensors 13b wird über ein an die Patientenmonitorschnittstelle 14b angeschlossenes Kabel 10b an den Patientenmonitor 15 ausgegeben.

Wie die Sensorvorrichtung 1a, so besitzt auch die Sensorvorrichtung 1b einen Speicherbaustein 18b, in welchem Patientendaten, Kalibrierdaten etc. gespeichert werden können, und ein Kennzeichnungsbauteil 20b, auf welchem eine Sensortypenkennung abgelegt ist, welche vom Patientenmonitor 15 abgefragt werden kann, um zu erkennen, um welche Sensorvorrichtung 1b es sich handelt. Ferner ist wiederum eine vom Patientenmonitor ansteuerbare Ausgabevorrichtung 22b vorgesehen. Die Sensorvorrichtung 1b besitzt an ihrer Rückseite ebenfalls einen aufgesteckten Halteplattenadapter 28b, über den sie werkzeugfrei an einer patientenbettseitigen Halteplatte angebracht und wieder werkzeugfrei von dieser gelöst werden kann.

Der Patientenmonitor 15 ist zur Auswertung des analogen Sensorsignals der Drucksensoren 13a und 13b ausgestattet. Dabei ist es gleichgültig, an welchen der Anschlüsse 31a, 31b der Sensorschnittstelle des Patientenmonitors 15 die Sensorkabel 10a, 10b jeweils angeschlossen werden, das der Patientenmonitor 15 anhand des jeweiligen Kennzeichnungssignals erkennt, von welcher Sensorvorrichtung 1a, 1b das Sensorsignal jeweils stammt.

Die Signalverarbeitung und Auswertung im Patientenmonitor 15 kann im Grunde so erfolgen, wie bei per se aus dem Stand der Technik bekannten Patientenmonitoren. So kann der Patientenmonitor beispielsweise zur Ausführung gängiger Pulskonturalgorithmen programmiert sein. Ferner kann der Patientenmonitor 15 weitere Anschlüsse 31c, 31d zum Anschließen weiterer Sensoren aufweisen und für die Auswertung weiterer Sensorsignale ausgestattet sein. Wie per se aus dem Stand der Technik bekannt, kann der Patientenmonitor 15 so für die Durchführung von (insbesondere transpulmonalen) Thermodilutionsmessungen, zur Verarbeitung von Plethysmographiesignalen, zur zentralvenösen Sauerstoffsättigungsmessung usw. ausgerüstet sein.

Aus den Sensorsignalen ermittelte physiologische Parameter, wie etwa das Herzzeitvolumen (Cardiac Output CO), cardio-pulmonale Volumen-Compartments etc., können über den Bildschirm 32 graphisch und numerisch angezeigt werden.

Die Katheter 3a, 3b können für die angedeuteten zusätzlichen Messaufgaben mit einem oder mehreren zusätzlichen Katheter-Ports 33a, 33b ausgestattet sein, wie per se aus dem Stand der Technik bekannt.

## Patentansprüche

1. System, aufweisend eine tragbare sterile Sensorvorrichtung (1a), welche folgendes aufweist:
ein elektronisches Sensorbauteil (13a) zum Messen eines physiologischen Druckes an einem Messort (4a) des Körpers eines Patienten,
eine Messortschnittstelle (6a) zum Anschließen des elektronischen Sensorbauteils (13a) an ein druckübertragendes Schlauchelement (2a) umfassende Messortverbindungsmittel zum Herstellen einer hydraulischen Verbindung als Messverbindung zwischen dem elektronischen Sensorbauteil (13a) und dem Messort (4a),
eine Patientenmonitorschnittstelle (14a) zum Anschließen des elektronischen Sensorbauteils (13a) an Patientenmonitorverbindungsmittel (10a) zum Herstellen einer Signalverbindung zwischen dem elektronischen Sensorbauteil (13a) und einem Patientenmonitor (15), und
ein Sensorgehäuse (12a) zum Unterbringen des elektronischen Sensorbauteils (13a), der Messortschnittstelle (6a) und der Patientenmonitorschnittstelle (14a),
wobei die Patientenmonitorschnittstelle (14a) einen Eingangskanal (17, 21) zum Empfangen eines Datensignals vom Patientenmonitor (15) aufweist,
die tragbare Sensorvorrichtung (1a) mindestens ein mittels des Datensignals beeinflussbares elektronisches Zusatzbauteil (18a, 22a) aufweist,
wobei es sich bei dem physiologischen Druck um einen Blutdruck handelt, das System einen zusätzlichen Drucksensor (9) und Zweitsensorverbindungsmittel (8) aufweist, **dadurch gekennzeichnet, dass** die Sensorvorrichtung (1a) eine Drucklinienschnittstelle (7) aufweist, an welche die Zweitsensorverbindungsmittel (8) angeschlossen sind, die eine hydraulische Verbindung zwischen dem an der Messortschnittstelle anschließbaren druckübertragenden Schlauchelement (2a) und dem zusätzlichen Drucksensor (9) herstellen, und
die Sensorvorrichtung (1a) leichter als 100 g ist.

2. System gemäß Anspruch 1, wobei das mindestens eine elektronische Zusatzbauteil (18a, 22a) einen Speicher (18a) zum Speichern von Patientendaten umfasst.

3. System gemäß einem der vorangehenden Ansprüche, wobei die Sensorvorrichtung (1a) eine optische und/oder akkustische Ausgabevorrichtung (22a) aufweist.

4. System gemäß einem der vorangehenden Ansprüche, wobei die Sensorvorrichtung (1a) in der Richtung Ihrer größten Ausdehnung kleiner als 100 Millimeter ist.

5. System gemäß einem der vorangehenden Ansprüche, wobei das Sensorgehäuse (12a) einen mechanischen Halterungsanschluss (28a) zum zeitweisen Befestigen des Sensorgehäuses (12a) an einer patientenbettseitigen Halterung aufweist.

6. System gemäß einem der vorangehenden Ansprüche, wobei die Patientenmonitorschnittstelle (14a) zum Herstellen einer analogen Signalverbindung (16) zwischen dem elektronischen Sensorbauteil (13a) und dem Patientenmonitor (15) zum Übermitteln eines analogen Sensorsignals eingerichtet ist,
die tragbare Sensorvorrichtung (1a) ein elektronisches Kennzeichnungsbauteil (20a) zum Bereitstellen eines Kennzeichnungssignals, welches eine Eigenschaft des durch die analoge Signalverbindung (16) zu übermittelnden Sensorsignals kennzeichnet, aufweist, und
die Patientenmonitorschnittstelle (14a) einen separaten Kennzeichnungskanal (19) zum Ausgeben des Kennzeichnungssignals an den Patientenmonitor (15) aufweist.

7. System gemäß einem der Ansprüche 1-5 ferner aufweisend einen Patientenmonitor (15), welcher eine Sensorschnittstelle zum Anschließen der Patientenmonitorverbindungsmittel (10a) zum Herstellen einer Signalverbindung zwischen der tragbaren Sensorvorrichtung (1a) und dem Patientenmonitor (15) aufweist,
wobei die Sensorschnittstelle einen Datenausgabekanal (17, 21) zum Ausgeben eines Datensignals durch die Patientenmonitorverbindungsmittel (10a) zur tragbaren Sensorvorrichtung (1a) aufweist.

8. System gemäß Anspruch 7, wobei die Patientenmonitorschnittstelle (14a) zum Herstellen einer analogen Signalverbindung (16) zwischen dem elektronischen Sensorbauteil (13a) und dem Patientenmonitor (15) zum Übermitteln eines analogen Sensorsignals eingerichtet ist,
die tragbare Sensorvorrichtung (1a) ein elektronisches Kennzeichnungsbauteil (20a) zum Bereitstellen eines Kennzeichnungssignals, welches eine Eigenschaft des durch die analoge Signalverbindung (16) zu übermittelnden Sensorsignals kennzeichnet, aufweist, und
die Patientenmonitorschnittstelle (14a) einen separaten Kennzeichnungskanal (19) zum Ausgeben des Kennzeichnungssignals an den Patientenmonitor (15) aufweist,
und wobei die Sensorschnittstelle zum Anschließen von Patientenmonitorverbindungsmitteln (10a) zum Herstellen der analogen Signalverbindung (16) zwischen einer tragbaren Sensorvorrichtung (1a) und dem Patientenmonitor (15) ausgebildet ist,
einen separaten Erkennungskanal (19) zum Einlesen des Kennzeichnungssignals in den Patientenmonitor (15) aufweist, und
der Patientenmonitor (15) dazu ausgebildet ist, das durch die analoge Signalverbindung (16) zu übermittelnde Sensorsignal in Abhängigkeit des Kennzeichnungssignals zu verarbeiten.

## Claims

1. A System, comprising a portable sterile sensor device (1a) that comprises the following:
an electronic sensor component (13a) for measuring a physiological pressure at a measurement site (4a) of the body of a patient,
a measurement site interface (6a) for connecting the electronic sensor component (13a) to measurement site connection means for establishing a hydraulic connection as a measurement connection between the electronic sensor component (13a) and the measurement site (4a), said measurement site connection means comprising a pressure transmitting hose element (2a),
a patient monitor interface (14a) for connecting the electronic sensor component (13a) to patient monitor connection means (10a) for establishing a signal connection between the electronic sensor component (13a) and a patient monitor (15), and
a sensor housing (12a) for accommodating the electronic sensor component (13a), the measurement site interface (6a), and the patient monitor interface (14a),
wherein the patient monitor interface (14a) has an input channel (17, 21) for receiving a data signal from the patient monitor (15),
the portable sensor device (1a) comprises at least one electronic additional component (18a, 22a) that can be influenced by the data signal,
wherein said physiological pressure is a blood pressure,
the system further comprises an additional pressure sensor (9) and second sensor connection means (8),
**characterized in that**
said sensor device (1a) comprises a pressure line interface (7) to which the second sensor connection means (8) are connected which establish a hydraulic connection between the pressure-transferring hose element (2a) connectable to said measurement site interface and the additional pressure sensor (9), and
the sensor device (1a) is lighter than 100 grams.

2. System according to claim 1, wherein the at least one electronic additional component (18a, 22a) comprises a memory (18a) for storing patient data.

3. System according to one of the preceding claims, wherein the sensor device (1a) comprises an optical and/or acoustical output device (22a).

4. System according to one of the preceding claims, wherein the sensor device (1a) is smaller than 100 millimeters in the direction of its greatest expanse.

5. System according to one of the preceding claims, wherein the sensor housing (12a) has a mechanical holding connector (28a) for temporarily affixing the sensor housing 12(a) to a holder at the patient's bedside.

6. System according to one of the preceding claims, wherein the patient monitor interface (14a) is adapted for establishing an analog signal connection (16) between the electronic sensor component (13a) and the patient monitor (15) for transmitting an analog sensor signal,
the portable sensor device (1a) comprises an electronic identification component (20a) for providing an identification signal, which identifies a property of the sensor signal to be transmitted by the analog signal connection (16), and
the patient monitor interface (14a) comprises a separate identification channel (19) for output of the identification signal to the patient monitor (15).

7. System according to one of claims 1-5 further comprising a patient monitor (15) which comprises a sensor interface for connecting the patient monitor connection means (10a) for establishing a signal connection between the portable sensor device (1a) and the patient monitor,
wherein the sensor interface comprises a data output channel (17, 21) for output of a data signal, via the patient monitor connection means (10a), to the portable sensor device (1a).

8. System according to claim 7, wherein the patient monitor interface (14a) is configured to establish an analog signal connection (16) between the electronic sensor component (13a) and the patient monitor (15) for transmitting an analog sensor signal,
the portable sensor device (1a) comprises an electronic identification component (20a) for providing an identification signal which identifies a property of the sensor signal to be transmitted by the analog signal connection (16), and
said patient monitor interface (14a) comprises a separate identification channel (19) for output of the identification signal to the patient monitor (15),
and wherein the sensor interface is adapted for connecting patient monitor connection means (10a) for establishing the analog signal connection (16) between the portable sensor device (1a) and the patient monitor (15),
the sensor interface comprises a separate recognition channel (19) for reading the identification signal into the patient monitor (15), and
the patient monitor (15) is adapted for processing the sensor signal to be transmitted by means of the analog signal connection (16) depending on the identification signal.

## Revendications

1. Système présentant un dispositif de capteur (1a) stérile portatif, lequel présente ce qui suit :
un composant de capteur (13a) électronique servant à mesurer une pression physiologique au niveau d'un emplacement de mesure (4a) du corps d'un patient,
une interface d'emplacement de mesure (6a) servant à raccorder le composant de capteur (13a) électronique à des moyens de liaison d'emplacement de mesure comprenant un élément de tuyau flexible (2a) de transfert de pression pour établir une liaison hydraulique en tant que liaison de mesure entre le composant de capteur (13a) électronique et l'emplacement de mesure (4a),
une interface de moniteur de patient (14a) servant à raccorder le composant de capteur (13a) électronique à des moyens de liaison de moniteur de patient (10a) pour établir une liaison de signaux entre le composant de capteur (13a) électronique et un moniteur de patient (15), et
un boîtier de capteur (12a) servant à abriter le composant de capteur (13a) électronique, l'interface d'emplacement de mesure (6a) et l'interface de moniteur de patient (14a),
dans lequel l'interface de moniteur de patient (14a) présente un canal d'entrée (17, 21) servant à recevoir un signal de données du moniteur de patient (15),
le dispositif de capteur (1a) portatif présente au moins un composant supplémentaire (18a, 22a) électronique pouvant être influencé au moyen du signal de données,
dans lequel la pression physiologique est une pression sanguine,
le système présente un capteur de pression (9) supplémentaire et des moyens de liaison de capteur secondaires (8), **caractérisé en ce que**
le dispositif de capteur (1a) présente une interface de ligne de pression (7), à laquelle les moyens de liaison de capteur secondaires (8) sont raccordés, qui établissent une liaison hydraulique entre l'élément de tuyau flexible (2a) de transfert de pression pouvant être raccordé à l'interface d'emplacement de mesure et le capteur de pression (9) supplémentaire, et
le dispositif de capteur (1a) présente un poids inférieur à 100 g.

2. Système selon la revendication 1, dans lequel l'au moins un composant supplémentaire (18a, 22a) électronique comprend une mémoire (18a) servant à mémoriser des données de patients.

3. Système selon l'une quelconque des revendications précédentes, dans lequel le dispositif de capteur (1a) présente un dispositif d'émission (22a) optique et/ou acoustique.

4. Système selon l'une quelconque des revendications précédentes, dans lequel le dispositif de capteur (1a) est dans le sens de son extension la plus grande inférieur à 100 millimètres.

5. Système selon l'une quelconque des revendications précédentes, dans lequel le boîtier de capteur (12a) présente un raccordement de fixation (28a) mécanique servant à fixer temporairement le boîtier de capteur (12a) au niveau d'une fixation côté lit du patient.

6. Système selon l'une quelconque des revendications précédentes, dans lequel l'interface de moniteur de patient (14a) est mise au point pour établir une liaison de signaux analogique (16) entre le composant de capteur (13a) électronique et le moniteur de patient (15) pour transmettre un signal de capteur analogique,
le dispositif de capteur (1a) portatif présente un composant d'identification (20a) électronique servant à fournir un signal d'identification, lequel identifie une propriété du signal de capteur à transmettre par la liaison de signaux (16) analogique, et
l'interface de moniteur de patient (14a) présente un canal d'identification (19) séparé servant à émettre le signal d'identification au moniteur de patient (15).

7. Système selon l'une quelconque des revendications 1 à 5,
présentant en outre un moniteur de patient (15), lequel présente une interface de capteur servant à raccorder les moyens de liaison de moniteur de patient (10a) pour établir une liaison de signaux entre le dispositif de capteur (1a) portatif et le moniteur de patient (15),
dans lequel l'interface de capteur présente un canal d'émission de données (17, 21) servant à émettre un signal de données par les moyens de liaison de moniteur de patient (10a) vers le dispositif de capteur (1a) portatif.

8. Système selon la revendication 7, dans lequel l'interface de moniteur de patient (14a) est mise au point pour établir une liaison de signaux (16) analogique entre le composant de capteur (13a) électronique et le moniteur de patient (15) pour transmettre un signal de capteur analogique,
le dispositif de capteur (1a) portatif présente un composant d'identification (20a) électronique servant à fournir un signal d'identification, lequel identifie une propriété du signal de capteur à transmettre par la liaison de signaux (16) analogique,
et
l'interface de moniteur de patient (14a) présente un canal d'identification (19) séparé servant à émettre le signal d'identification au moniteur de patient (15),
et dans lequel l'interface de capteur est réalisée pour raccorder des moyens de liaison de moniteur de patient (10a) pour établir la liaison de signaux (16) analogique entre un dispositif de capteur (1a) portatif et le moniteur de patient (15),
un canal d'identification (19) séparé servant à lire le signal d'identification dans le moniteur de patient (15), et
le moniteur de patient (15) est réalisé pour traiter le signal de capteur à transmettre par la liaison de signaux (16) analogique en fonction du signal d'identification.
